# EUROPÄISCHE PATENTSCHRIFT

(11) **EP 2 841 169 B1**
(45) Veröffentlichungstag und Bekanntmachung des Hinweises auf die Patenterteilung: **04.04.2018**
(21) Anmeldenummer: 13706029.9
(22) Anmeldetag: 27.02.2013
(51) Int. Cl.: A61Q 5/12, A61K 8/81, A61K 8/898

(54) **HAARBEHANDLUNGSMITTEL MIT HYDROXY-TERMINIERTEN ORGANOPOLYSILOXAN(EN) UND VERDICKUNGSMITTEL(N)**
HAIR PREPARATIONS COMPRISING HYDROXY-TERMINATED ORGANOPOLYSILOXANE(S) AND THICKENER(S)
PRODUIT DE TRAITEMENT CAPILLAIRE CONTENANT UN (DES) ORGANOPOLYSILOXANE(S) À TERMINAISON HYDROXY ET UN (DES) AGENT(S) ÉPAISSISSANT(S)

(30) Priorität: 26.04.2012 DE 102012206948
(43) Veröffentlichungstag der Anmeldung: 04.03.2015
(73) Patentinhaber: Henkel AG & Co. KGaA, 40589 Düsseldorf (DE)
(72) Erfinder: SCHULZE ZUR WIESCHE, Erik, 20144 Hamburg (DE); SEMRAU, Markus, 40764 Langenfeld (DE)
(86) Internationale Anmeldenummer: PCT/EP2013/053876
(87) Internationale Veröffentlichungsnummer: WO 2013/159964

(56) Entgegenhaltungen:
- DE-A1-102010 041 503

## Beschreibung

Die Erfindung betrifft Haarbehandlungsmittel, die speziell substituierte(s) Silikon(e) enthalten sowie die Verwendung dieser Mittel zur Reinigung und/oder Pflege von Haaren.

Pflegemittel für keratinische Fasern beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein. Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngerer Zeit so genannte Kombinationspräparate entwickelt, um den Aufwand der üblichen mehrstufigen Verfahren, insbesondere bei der direkten Anwendung durch Verbraucher, zu verringern. Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in tensid- und/oder elektrolythaltigen Formulierungen besteht Bedarf an zusätzlichen pflegenden Wirkstoffen, die sich problemlos in bekannte Formulierungen einarbeiten lassen und dort nicht aufgrund von Unverträglichkeiten mit anderen Inhaltsstoffen in ihrer Wirkung abgeschwächt werden.

Silikone und unter ihnen aminofunktionelle Silikone sind als Pflegestoffe in Haarbehandlungsmitteln bekannt, und entsprechende Produkte sind im Markt weit verbreitet. Es besteht aber weiterhin der Bedarf, die erzielten Effekte, insbesondere im Hinblick auf den Griff, die Kämmbarkeit, die Weichheit und das Volumen der Haare bzw. der Frisur zu verbessern und die Einsatzmengen zu verringern.

Der vorliegenden Erfindung lag die Aufgabe zugrunde, silikonhaltige Haarbehandlungsmittel bereitzustellen, die den mit ihnen behandelten Haaren noch bessere Eigenschaften verleihen als Haarbehandlungsmittel mit bekannten Amodimethiconen. Darüber hinaus sollten auch bei deutlich verringerten Einsatzmengen gleich gute oder bessere Effekte erzielt werden können. Insbesondere sollten die Produkte den Griff, die Kämmbarkeit, die Weichheit und das Volumen der Haare bzw. der Frisur verbessern und den Kontaktwinkel von Wassertropfen, die auf die behandelten Haare kommen, deutlich minimieren, was ein Maß für die Produktleistung ist.

Die DE 10 2010 041 503A1 offenbart in Beispiel 5 kosmetische Mittel, welche Guar Hydroxypropyltromoniumchlorid und ein Hydroxy-terminiertes Organopolysiloxan enthalten. Der Einsatz anderer Verdickungsmittel wird dort nicht erwähnt.

Es wurde nun gefunden, daß besonders vorteilhafte Ergebnisse erzielt werden, wenn man bestimmte Silikon(e) und Konditioniermittel in Haarbehandlungsmittel inkorporiert.
Ein erster Gegenstand der vorliegenden Erfindung ist eine kosmetische Zusammensetzung, enthaltend
(a) mindestens ein Verdickungsmittel, ausgewählt aus assoziativen Verdickungsmitteln
(b) mindestens ein hydroxy-terminiertes Organopolysiloxan der allgemeinen Formel (I), 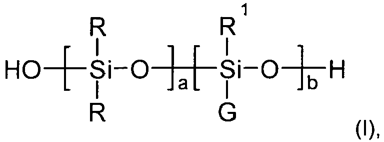 wobei
   - R einen einwertigen unsubstituierten oder halogensubstituierten Kohlenwasserstoffrest mit 1 bis 20 Kohlenstoffatomen,
   - R¹ einen einwertigen unsubstituierten oder halogensubstituierten Kohlenwasserstoffrest mit 1 bis 20 Kohlenstoffatomen, -OR⁴ oder -OH,
   - R⁴ einen Alkylrest mit 1 bis 6 Kohlenstoffatomen,
   - G eine Gruppe der allgemeinen Formel (II) 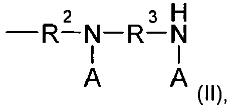 wobei
   - R², R³ unabhängig voneinander einen zweiwertigen Kohlenwasserstoffrest mit 1 bis 6 Kohlenstoffatomen, wobei nicht benachbarte -CH₂-Einheiten ersetzt sein können durch Einheiten, die ausgewählt werden aus -C(=O)-, -O-, und -S-,
   - A R⁵-C(=O)-,
   - R⁵ einen Alkylrest mit 1 bis 20 Kohlenstoffatomen,
   - a ganzzahlige Werte von 100 bis 1500 und
   - b ganzzahlige Werte von mindestens 1 bedeuten.

Das erfindungsgemäße Mittel ist ein kosmetisches Mittel. Je nach Anwendungsgebiet unterscheidet man daher ein breite Palette kosmetischer Mittel, beispielsweise zur Hautpflege (Badepräparate, Hautwasch- u. -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- u. Mundpflege (Zahn- und Mundpflegemittel, Gebißpflegemittel, Prothesenhaftmittel) und solche zur Haarpflege (Haarwaschmittel, Haarpflegemittel, Haarverfestigungsmittel, Haarverformungsmittel, Mittel zur Farbänderung).

Erfindungsgemäß bevorzugte kosmetische Mittel sind ausgewählt aus der Gruppe der Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Besonders bevorzugte erfindungsgemäße kosmetische Mittel dienen der Behandlung keratinischer Fasern und stellen somit Haarbehandlungsmittel dar. Haarbehandlungsmittel im Sinne der vorliegenden Erfindung sind beispielsweise Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Im Hinblick auf die Tatsache, daß Männer oft die Anwendung mehrerer unterschiedlicher Mittel und/oder mehrere Anwendungsschritte scheuen, sind erfindungsgemäße Mittel bevorzugt solche Mittel, die der Mann ohnehin anwendet. Bevorzugte erfindungsgemäße Mittel sind daher Shampoos, Konditioniermittel oder Haar-Tonics.

Die Zusammensetzungen der Erfindung weisen verbesserte kosmetische Eigenschaften auf (bei Haaren beispielsweise Leichtigkeit, Weichheit, Entwirrbarkeit, natürliches Gefühl und luftige Frisur, Helligkeit), zudem sind die Effekte persistenter und anhaltend. Insbesondere sind diese Effekte beständig gegen viele Shampoos.

Darüber hinaus führen die Zusammensetzungen der Erfindung bei Applikation auf die Haut (beispielsweise durch ein Schaumbad oder Duschgel), zu einer verbesserten Geschmeidigkeit der Haut.

Die erfindungsgemäßen Mittel enthalten als ersten wesentlichen Inhaltsstoff mindestens ein hydroxy-terminiertes Organopolysiloxan der allgemeinen Formel (I), 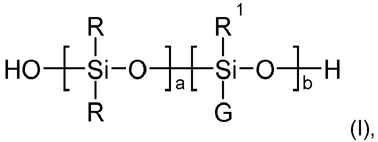 wobei
- R einen einwertigen unsubstituierten oder halogensubstituierten Kohlenwasserstoffrest mit 1 bis 20 Kohlenstoffatomen,
- R¹ einen Rest R, -OR⁴ oder -OH,
- R⁴ einen Alkylrest mit 1 bis 6 Kohlenstoffatomen,
- G eine Gruppe der allgemeinen Formel (II) 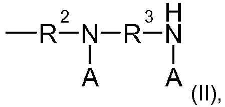 wobei
- R², R³ unabhängig voneinander einen zweiwertigen Kohlenwasserstoffrest mit 1 bis 6 Kohlenstoffatomen, wobei nicht benachbarte -CH₂-Einheiten ersetzt sein können durch Einheiten, die ausgewählt werden aus -C(=O)-, -O-, und -S-,
- A R⁵-C(=O)-,
- R⁵ einen Alkylrest mit 1 bis 20 Kohlenstoffatomen,
- a ganzzahlige Werte von 100 bis 1500 und
- b ganzzahlige Werte von mindestens 1 bedeuten.

Die einzelnen Silioxaneinheiten a und b in Formel (I) sowie allen nachfolgenden Formeln können als Blockcopolymer oder statistisch im Molekül verteilt vorliegen, vorzugsweise sind sie statistisch verteilt.

Mit den Definitionen für G und A ergibt sich, daß erfindungsgemäße Mittel mindestens ein hydroxy-terminiertes Organopolysiloxan der allgemeinen Formel (la) enthalten: 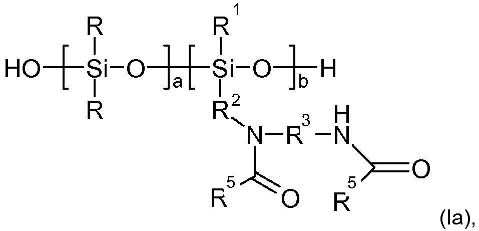 in der R, R¹, R², R³, R⁵, a und b wie oben definiert sind.

Der Rest R¹ kann für einen einwertigen unsubstituierten oder halogensubstituierten Kohlenwasserstoffrest mit 1 bis 20 Kohlenstoffatomen, eine OH-Gruppe oder einen Rest OR⁴ stehen, wobei R⁴ seinerseits für einen Alkylrest mit 1 bis 6 Kohlenstoffatomen steht. Bevorzugte Reste R¹ sind Methyl-, Ethyl-, n-Propyl, Isopropyl-, n-Butyl-, Isobutyl-, sec-Butyl-, tert-Butyl, n-Pentyl, n-Hexyl-, Hydroxy-, Methoxy-, Ethoxy-, n-Propoxy- und iso-Propoxy-Reste.

Besonders bevorzugte erfindungsgemäße Mittel enthalten mindestens ein hydroxy-terminiertes Organopolysiloxan der allgemeinen Formel (Ia-1) und/oder (la-2) und/oder (la-3) und/oder (la-4) und/oder (la-5) und/oder (la-6) und/oder (la-7) und/oder (la-8) und/oder (la-9) wie im Prioritätsdokument auf den Seiten 6 und 7 offenbart.

In besonders bevorzugten erfindungsgemäßen kosmetischen Zusammensetzungen ist R ausgewählt aus Methyl-, Ethyl-, n-Propyl, Isopropyl-, n-Butyl-, Isobutyl-, sec-Butyl-, tert-Butyl, n-Pentyl, n-Hexyl-, Phenyl-Resten, wobei R äußerst bevorzugt für Methyl, Ethyl oder Phenyl steht. Für R = Methyl wird Formel (la) zu Formel (Ib), für R = Ethyl zu Formel (Ic), für R = Phenyl zu Formel (Id), so daß besonders bevorzugte erfindungsgemäße Mittel mindestens ein hydroxy-terminiertes Organopolysiloxan der allgemeinen Formel (Ib-1) und/oder (Ib-2) und/oder (Ib-3) und/oder (Ib-4) und/oder (Ib-5) und/oder (Ib-6) und/oder (Ib-7) und/oder (Ib-8) und/oder (Ib-9) und/oder (Ic-1) und/oder (Ic-2) und/oder (Ic-3) und/oder (Ic-4) und/oder (Ic-5) und/oder (Ic-6) und/oder (Ic-7) und/oder (Ic-8) und/oder (Ic-9) und/oder (Id-1) und/oder (Id-2) und/oder (Id-3) und/oder (Id-4) und/oder (Id-5) und/oder (Id-6) und/oder (Id-7) und/oder (Id-8) und/oder (Id-9) wie im Prioritätsdokument auf den Seiten 8 bis 13 offenbart, enthalten.

Bevorzugte erfindungsgemäße kosmetische Zusammensetzungen enthalten mindestens ein hydroxy-terminiertes Organopolysiloxan der allgemeinen Formel (I), in der die Reste R², R³ unabhängig voneinander ausgewählt sind aus Ethylen-, n-Propylen-, iso-Butylen- oder n-Butylenresten.

Ganz besonders bevorzugt steht R² für einen n-Propylenrest und R³ gleichzeitig für einen Ethylenrest, so daß die Gruppierung G vorzugsweise eine Gruppierung 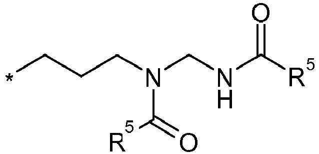 ist.

Ganz besonders bevorzugte erfindungsgemäße Mittel enthalten demnach mindestens ein hydroxy-terminiertes Organopolysiloxan der allgemeinen Formel (Ie-1) und/oder (Ie-2) und/oder (Ie-3) und/oder (Ie-4) und/oder (Ie-5) und/oder (Ie-6) und/oder (Ie-7) und/oder (Ie-8) und/oder (Ie-9) mit den Definitionen für R wie bei Formel (I) angegeben, wobei besonders bevorzugte R für Methyl-, Ethyl- oder Phenylreste stehen:

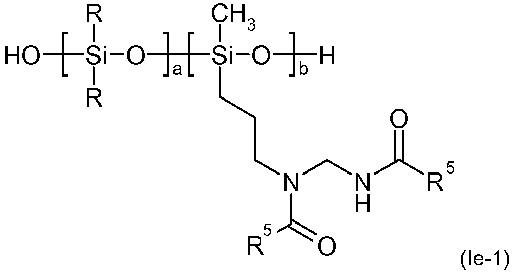

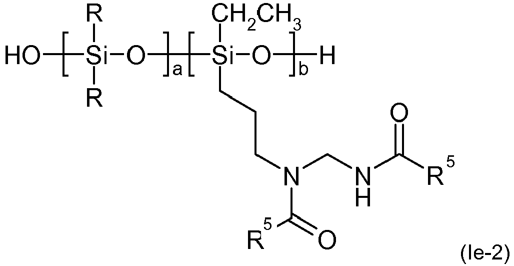

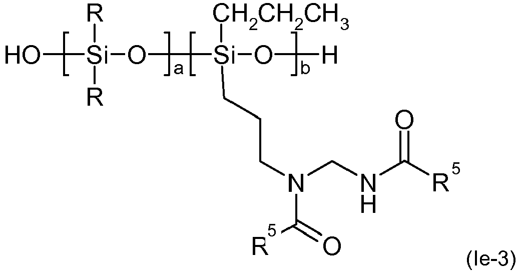

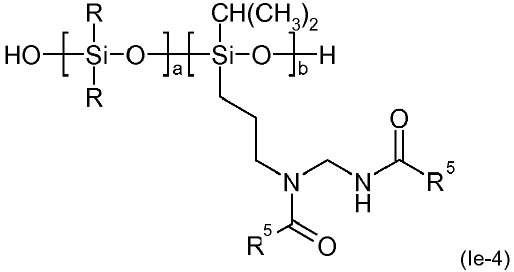

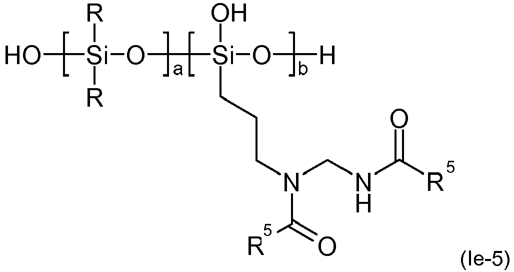

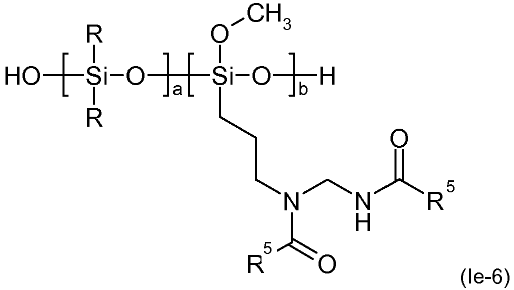

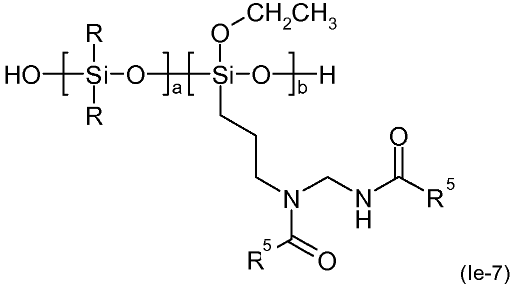

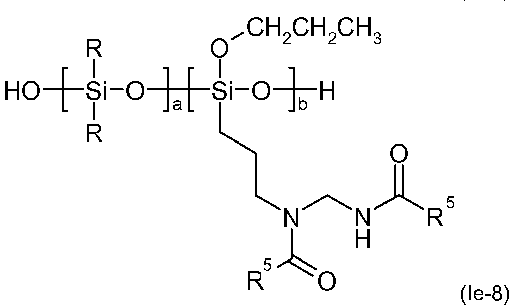

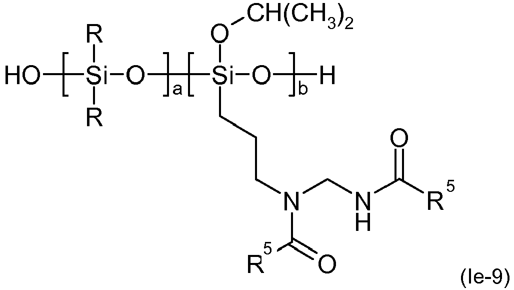

Ganz besonders bevorzugte erfindungsgemäße kosmetische Zusammensetzung sind dadurch gekennzeichnet, daß sie mindestens ein hydroxy-terminiertes Organopolysiloxan der allgemeinen Formel (I) enthalten, in der R⁵ ausgewählt ist aus Methyl-, Ethyl-, n-Propyl, Isopropyl-, n-Butyl-, Isobutyl-, sec-Butyl-, tert-Butyl, n-Pentyl, n-Hexyl-Resten, wobei R⁵ äußerst bevorzugt für Methyl, Ethyl, n-Propyl oder Isopropyl steht.

Für R⁵ = Methyl wird Formel (Ie) zu Formel (If), für R⁵ = Ethyl zu Formel (Ig), für R⁵ = n-Propyl zu Formel (Ih), für R⁵ = Isopropyl zu Formel (Ii), so daß besonders bevorzugte erfindungsgemäße Mittel mindestens ein hydroxy-terminiertes Organopolysiloxan der allgemeinen Formel (If-4) und/oder (If-5) und/oder (If-6) und/oder (If-7) und/oder (If-8) und/oder (If-9) und/oder (Ig-1) und/oder (Ig-2) und/oder (Ig-3) und/oder (Ig-4) und/oder (Ig-5) und/oder (Ig-6) und/oder (Ig-7) und/oder (Ig-8) und/oder (Ig-9) und/oder (Ih-1) und/oder (Ih-2) und/oder (Ih-3) und/oder (Ih-4) und/oder (Ih-5) und/oder (Ih-6) und/oder (Ih-7) und/oder (Ih-8) und/oder (Ih-9) und/oder (Ii-1) und/oder (Ii-2) und/oder (Ii-3) und/oder (Ii-4) und/oder (Ii-5) und/oder (Ii-6) und/oder (Ii-7) und/oder (Ii-8) und/oder (Ii-9) wie im Prioritätsdokument auf den Seiten 16 bis 25 offenbart, enthalten.

In allen genannten Formeln sind die Vertreter, in den R für einen Methylrest steht, besonders bevorzugt.

In allen genannten Formeln steht der Index a für ganzzahlige Werte von 100 bis 1500. Vorzugsweise bedeutet a ganzzahlige Werte von mindestens 200, insbesondere mindestens 500 und höchsten 1300, besonders bevorzugt höchstens 1100 und insbesondere höchstens 900. Erfindungsgemäße Mittel, in denen a Werte von 220 bis 910 annimmt, sind besonders bevorzugt. In allen genannten Formeln steht der Index b für ganzzahlige Werte von mindestens 1. Vorzugsweise bedeutet b ganzzahlige Werte von höchstens 100, insbesondere höchstens 50, bevorzugt höchstens 10 und insbesondere höchstens 5. Erfindungsgemäße Mittel, in denen b die Werte 1, 2, 3, 4 oder 5 annimmt, sind besonders bevorzugt.

Vorzugsweise werden a und b so gewählt, daß das Organopolysiloxan der Formel (I) bei Normalbedingungen (20°C, 1013,25 mbar) eine Viskosität (Brookfield RTV, Spindel 4, 20 U/min) von mindestens 100 mPas, vorzugsweise mindestens 1000 mPas, weiter bevorzugt mindestens 5000 mPas und insbesondere mindestens 15.000 mPas aufweist. Vorzugsweise beträgt die Viskosität maximal 500.000 mPas, vorzugsweise höchstens 200.000 mPas, besonders bevorzugt maximal 100.000 mPas und insbesondere höchstens 60.000 mPas. Erfindungsgemäße Mittel, in denen das Organopolysiloxan der Formel (I) Viskositäten von 17.000 bis 55.000 mPas aufweist, sind besonders bevorzugt.

Vorzugsweise beträgt die Aminzahl des Organopolysiloxans der Formel (I) mindestens 0,001 mmol/g, besonders bevorzugt mindestens 0,01 mmol/g. Besonders bevorzugt ist die Aminzahl höchstens 5 mmol/g, weiter bevorzugt höchstens 1 mmol/g, noch weiter bevorzugt höchstens 0,1 mmol/g und insbesondere maximal 0,05 mmol/g. Erfindungsgemäße Mittel, in denen das Organopolysiloxan der Formel (I) Aminzahlen von 0,015 bis 0,045 mmol/g aufweist, sind besonders bevorzugt.

Das bzw. die Organopolysiloxan(e) der Formel (I) können je nach Anwendungszweck der erfindungsgemäßen Mittel in variierenden Mengen eingesetzt werden. Bevorzugte erfindungsgemäße Kosmetische Mittel sind dadurch gekennzeichnet, daß sie - bezogen auf ihr Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% Organopolysiloxan(e) der Formel (I) enthalten.

Es hat sich gezeigt, daß die Wirkung der erfindungsgemäß eingesetzten Silikone noch gesteigert werden kann, wenn bestimmte nichtionische Komponenten ebenfalls in den erfindungsgemäßen Mitteln eingesetzt werden. Zudem haben diese nichtionischen Komponenten positive Effekte auf die Lagerstabilität der erfindungsgemäßen Mittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die erfindungsgemäßen Mittel inkorporiert werden. Erfindungsgemäß besonders bevorzugte kosmetische Zusammensetzungen enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen.

Erfindungsgemäß bevorzugte Organopolysiloxan(e) der Formel (I) weisen sowohl Hydroxy- als auch Alkoxygruppen auf. Erfindungsgemäß besonders bevorzugte kosmetische Zusammensetzungen enthalten Organopolysiloxan(e) der Formel (I), in denen das Molverhältnis Hydroxy/Alkoxy im Bereich von 0,2:1 bis 0,4:1, vorzugsweise im Bereich von 1:0,8 bis 1:1,1 liegt.

Die mittleren Molekulargewichte der Organopolysiloxan(e) der Formel (I) betragen vorzugsweise von 2.000 bis 200.000 und noch mehr bevorzugt von 5.000 bis 100.000, insbesondere 10.000 bis 50.000 Dalton. Kosmetische Zusammensetzungen, bei denen die gewichtmittlere Molmasse der in ihnen enthaltenen Organopolysiloxan(e) der Formel (I) im Bereich von 2.000 bis 1.000.000 gmol⁻¹, vorzugsweise im Bereich von 5.000 bis 200.000 gmol⁻¹ liegt, sind bevorzugt.

Die mittleren Molekulargewichte von aminosubstituierten Silikonen sind beispielsweise durch Gelpermeationschromatographie (GPC) bei Raumtemperatur in Polystyrol messbar. Als Säulen können Styragel Spalten µ, als Eluent THF und als Flussrate 1 ml / min gewählt werden. Die Detektion erfolgt vorzugsweise mittels Refraktometrie und UV-Meter. Der Einsatz der Organopolysiloxan(e) der Formel (I) erfolgt vorzugsweise als Öl in Wasser-Emulsion. Die Öl in Wasser-Emulsion kann ein oder mehrere Tenside enthalten. Die Tenside können von beliebiger Art sein, bevorzugt kationische und / oder nichtionische. Vorzugsweise liegt die zahlenmittlere durchschnittliche Größe der Silicon-Tröpfchen in der Emulsion zwischen 3 nm und 500 nm, besonders bevorzugt zwischen 5 nm und 60 nm (inklusive) und insbesondere zwischen 10 nm und 50 nm (inklusive).

Erfindungsgemäße kosmetische Zusammensetzungen, bei denen das/die Organopolysiloxan(e) der Formel (I) in Form einer Öl-in-Wasser-Emulsion vorliegen, in der die zahlenmittlere Größe der Siliconpartikel in der Emulsion im Bereich von 3 bis 500 nm, vorzugsweise im Bereich von 5 bis 60 nm liegt, sind erfindungsgemäß bevorzugt.

Ein weiterer wesentlicher Bestandteil der erfindungsgemäßen Zusammensetzungen ist ein Verdickungsmittel, ausgewählt aus assoziativen Verdickungsmitteln. Dessen Funktion es ist, die Viskosität der Zusammensetzung zu erhöhen.

"Assoziative Verdickungsmittel" nach der Erfindung sind Verdickungsmittel mit sowohl hydrophilen als auch hydrophoben Einheiten, insbesondere mit mindestens einer Fettkette C₈-C₃₀ und mindestens einer hydrophilen Einheit.

Erfindungsgemäß bevorzugte kosmetische Zusammensetzungen enthalten als assoziatives Verdickungsmittel ein assoziatives Polymer ist, das ausgewählt ist unter
(i.) nichtionischen amphiphilen Polymeren, die mindestens ein Fettkette und mindestens eine hydrophile Einheit enthalten;
(ii.) anionischen amphiphilen Polymeren, die mindestens eine hydrophile Einheit und mindestens eine Einheit mit Fettkette enthalten;
(iii.) kationischen amphiphilen Polymeren, die mindestens eine hydrophile Einheit und mindestens eine Einheit mit Fettkette enthalten;
(iv.) amphoteren amphiphilen Polymeren, die mindestens eine hydrophile Einheit und mindestens eine Einheit mit Fettkette enthalten,
wobei die Fettketten 10 bis 30 Kohlenstoffatome aufweisen.

Das nicht-ionische amphiphile Polymere mit mindestens einer Fettsäure Kette und mindestens einer hydrophilen Einheit ist vorzugsweise ausgewählt aus:
(1) Cellulosen, die mit Gruppen modifiziert sind, die mindestens eine Fettkette aufweisen;
(2) Hydroxypropylguarverbindungen, die mit Gruppen modifiziert sind, die mindestens eine Fettkette aufweisen;
(3) Urethanpolyether, die mindestens eine Fettkette enthalten, wie Alkyl- oder Alkenylgruppen mit 10 bis 30 Kohlenstoffatomen;
(4) Copolymeren von Vinylpyrrolidon und hydrophoben Monomeren mit Fettkette;
(5) Copolymeren von Alkyl(C₁₋₆)methacrylaten oder Alkyl(C₁₋₆)-acrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten.

Beispiele für (1) sind Hydroxyethylcellulosen mit Gruppen, die mindestens eine Fettkette, wie Alkyl, Arylalkyl, Alkylaryl, oder Mischungen davon enthalten, wobei die Alkylgruppen vorzugsweise ausgewählt sind aus C₈-C₂₂, wie das Produkt Natrosol Plus Grade 330 CS (Alkyl C16) von der Firma Aqualon, oder das Produkt Bermocoll EHM 100 von der Firma Berol Nobel, oder mit Polyalkylenglykolethergruppen versehene Alkylphenole, wie das Produkt AMERCELL POLYMER HM-1500 (Polyethylenglykol (15) Nonylphenolethersulfats) von Amerchol.

Beispiele für (2) sind das Produkt Esaflor HM 22 (C22-Alkylkette) von der Firma Lamberti, MiraCare XC95-3 (C14-Alkylkette) und RE205-1 (C20-Alkylkette) von Rhodia Chimie.

Beispiele für (3) sind die Produkte Dapral T 210 und T 212 Dapral von Akzo oder die Produkte Aculyn Aculyn 44 und 46 von Rohm & Haas.

Beispiele für (4) sind Antaron V216 oder V216 GANEX (Vinylpyrrolidon / Hexadecen-Copolymer) von ISP und Antaron V220 oder V220 GANEX (Vinylpyrrolidon / Eicosen) von ISP.

Ein Beispiel für (5) ist das oxyethylenierte Copolymer aus Methylmethacrylat / Stearylacrylat, das von Goldschmidt unter der Bezeichnung Antil 208 vertrieben wird.

Ein Beispiel für (6) ist das Copolymer von Polyethylenglykol-methacrylat / Laurylmethacrylat. Erfindungsgemäß bevorzugte kosmetische Zusammensetzungen sind daher dadurch gekennzeichnet, daß die nichtionischen amphiphilen Polymere, die mindestens eine Fettkette und mindestens eine hydrophile Einheit enthalten, ausgewählt sind unter
(1) Cellulosen, die mit Gruppen modifiziert sind, die mindestens eine Fettkette aufweisen;
(2) Hydroxypropylguarverbindungen, die mit Gruppen modifiziert sind, die mindestens eine Fettkette aufweisen;
(3) Urethanpolyether, die mindestens eine Fettkette enthalten, wie Alkyl- oder Alkenylgruppen mit 10 bis 30 Kohlenstoffatomen;
(4) Copolymeren von Vinylpyrrolidon und hydrophoben Monomeren mit Fettkette;
(5) Copolymeren von Alkyl(C₁₋₆)_{M}ethacrylaten oder Alkyl(C₁₋₆)-acrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten.

Unter den anionischen amphiphilen Polymere der Erfindung mit mindestens einem hydrophilen und mindestens einer Fettkette sind solche mit mindestens einer Allylether-Einheit mit einer Fettkette und mindestens einer hydrophilen Einheit aus einem ethylenisch ungesättigten anionischen Monomer, insbesondere Acrylsäure, Methacrylsäure oder deren Mischungen bevorzugt. Besonders bevorzugt sind Allylether mit eine Fettkette entsprechend dem Monomer der Formel (M-III):

CH₂ = C(R1)CH₂-OBₙR (M-III)

wobei R1 -H oder -CH₃ ist, B eine Ethylenoxydgruppierung bezeichnet, n Null oder eine ganze Zahl zwischen 1 bis 100 bedeutet, R einen Kohlenwasserstoffrest, ausgewählt aus Alkyl, Arylalkyl, Aryl, Alkylaryl, Cycloalkyl mit 10 bis 30 C-Atomen, vorzugsweise 10 bis 24 und noch mehr vor allem 12 bis 18 Kohlenstoffatomen darstellt.

Besonders bevorzugte Einheiten der Formel (M-III) sind solche, in denen R1 = -H, n = 10, und R ein Stearylrest (C18) ist.

Unter diesen anionischen amphiphilen Polymere, sind besonders bevorzugt Acrylate mit 20 bis 60 Gew.% Acrylsäure und/oder Methacrylsäure, 5 bis 60 Gew.% Niederalkyl-(Meth-)Acrylaten, von 2 bis 50 Gew.% Allylether mit einer Fettkette der Formel (M-III) und 0 bis 1 Gew.% Vernetzer. Handelsüblich ist beispielsweise ein Copolymer aus Diallylphthalat, (Meth)Acrylat-, Allyl-Divinylbenzoldimethacrylat, (Poly) Ethylenglykol und Methylen-bis-Acrylamid.

Besonders bevorzugte Terpolymere von Methacrylsäure, Ethylacrylat, Polyethylenglykol (10 EO) Stearylalkoholether (Steareth 10) werden unter den Namen SALCARE SC 80 und SALCARE SC90 verkauft, die 30%-ige wässrige Emulsionen von einem vernetzten Terpolymer aus Methacrylsäure, Ethylacrylat und Steareth-10-Allylether (40/50/10) sind.

Bevorzugte erfindungsgemäße kosmetische Zusammensetzungen sind dadurch gekennzeichnet, daß die anionischen amphiphilen Polymere, die mindestens eine hydrophile Einheit und mindestens eine Einheit mit Fettkette enthalten, unter den Verbindungen, die mindestens eine Allylethereinheit mit Fettkette und mindestens eine hydrophile Einheit enthalten, die aus einem ethylenisch ungesättigten, anionischen Monomer aufgebaut ist, Verbindungen, die mindestens eine hydrophile Einheit vom Typ einer olefinisch ungesättigten Carbonsäure und mindestens eine hydrophobe Einheit ausschließlich vom Typ eines Alkyl(C₁₋₃₀)esters einer ungesättigten Carbonsäure enthalten, und Copolymeren Methacrylsäure/ Methylacrylat/ Dimethyl-meta-isopropenyl-benzylisocyanat eines ethoxylierten Alkohols ausgewählt sind.

Die anionischen amphiphilen Polymere können weiter ausgewählt werden aus denjenigen, die mindestens eine hydrophilen Einheit von olefinisch ungesättigten Carbonsäuren und mindestens eine hydrophobe Einheit des Typs (C₁₀-C₃₀)-Alkylester einer ungesättigten Carbonsäure enthalten. Die hydrophile Einheit vom olefinisch ungesättigten Carbonsäure-Typ entspricht zum Beispiel dem Monomer der Formel (M-IV):

H₂C=C(R1)C(O)-OH (M-IV)

in denen R1 aus -H, -CH₃ und -C₂H₅ gewählt wird, i.e. Acrylsäure-, Methacrylsäure- und Ethacrylsäure-Einheiten. Und die hydrophobe Einheit vom Typ der (C₁₀-C₃₀) Alkylester einer ungesättigten Carbonsäure entspricht, zum Beispiel dem Monomer der Formel (V):

H₂C=C(R1)C(O)-OR2 (M-V)

in denen R1 aus -H, -CH₃ und -C₂H₅ gewählt wird (d.h. Acrylat-, Methacrylat- und Ethacrylat-Einheiten), R2 von C₁₀-C₃₀ Alkylreste gewählt, zum Beispiel C₁₂-C₂₂-Alkylrest.

Beispiele für (C₁₀-C₃₀) Alkylester von ungesättigten Carbonsäuren sind Laurylacrylat, Stearylacrylat Decylacrylat, Isodecylacrylat und Dodecylacrylat, und die entsprechenden Methacrylate, Laurylmethacrylat, Stearylmethacrylat, Decylmethacrylat, Isodecylmethacrylat und Dodecylmethacrylat.

Repräsentative anionische amphiphile Polymere, die verwendet werden können, sind auch Polymere aus einer Mischung von Monomeren, umfassend
(i) Acrylsäureester der Formel (M-VI)

   H₂C=C(R1)C(O)-OR2 (M-VI)

   in denen R 1 aus -H, -CH₃ gewählt wird (d.h. Acrylat-, Methacrylat-Einheiten), R2 von C₁₀-C₃₀ Alkylreste gewählt, zum Beispiel C₁₂-C₂₂-Alkylrest und einem Vernetzungsmittel; wie Polymere von 95% bis 60 Gew.% der Acrylsäure (hydrophile Einheit), 4% bis 40 Gew.% C10-C30 Alkylacrylat (hydrophobe Einheit) und 0% bis 6 Gew.% Vernetzung polymerisierbarer Monomere oder Polymere abgeleitet von 98% auf 96% des Gewichts der Acrylsäure (hydrophile Einheit), 1% bis 4 Gew.% C10-C30 Alkylacrylat (hydrophobe Einheit) und 0,1% bis 0,6 Gew.% Vernetzung polymerisierbarer Monomere oder
(ii) Acrylsäure und Laurylmethacrylat, wie die Polymere, die aus 66 Gew.% Acrylsäure und 34 Gew.% Laurylmethacrylat gebildet werden.

Das Vernetzungsmittel kann ein Monomer sein, das eine Gruppe mit mindestens einer anderen polymerisierbaren Gruppe enthält, deren ungesättigten Bindungen nicht konjugiert zueinander sind. Genannt seien zum Beispiel der Polyallylethern wie Polyallylsucrose und Polyallylpentaerythritol. Unter den oben erwähnten Polymeren sind beispielsweise die Produkte der Firma Goodrich unter den Handelsnamen Pemulen TR1, Pemulen TR2, Carbopol 1382 und weitere, zum Beispiel, Pemulen TR1 und das Produkt Coatex SX von Seppic bevorzugt.

Unter anionischen amphiphilen Fettsäuren-Kette Polymere seien auch die Methacrylsäure / Methylacrylat / ethoxylierte Alkyl Dimethyl-meta-isopropenylbenzylisocyanate Copolymer genannt, die unter dem Namen Viscophobe DB 1000 von der Firma Amerchol vertrieben werden.

Es können auch kationische amphiphile Polymere verwendet werden, zum Beispiel aus quaternisierten Cellulose-Derivaten und Polyacrylaten mit Aminoseitengruppen.

Die quaternisierte Cellulose-Derivate sind zum Beispiel von ausgewählten quaternisierten Cellulosen mit Gruppen, die mindestens eine Fettkette, wie Alkyl, Arylalkyl und Alkylarylgruppen mit mindestens 8 C-Atomen und deren Mischungen gebildet. Quaternisiert Hydroxyethylcellulosen mit Gruppen, die mindestens eine Fettkette, wie Alkyl, Arylalkyl und Alkylarylgruppen mit mindestens 8 C-Atomen und deren Mischungen sind bevorzugt. Quaternierte und nichtquaternisierte Polyacrylate mit Aminoseitengruppen und zum Beispiel hydrophoben Gruppen, wie Steareth 20 (Polyoxy-ethylenated (20) Stearylalkohol) und (C10-C30)-Alkyl PEG-20 itaconate sind ebenfalls bevorzugt.

Die Alkylreste der oben genannten quaternisierten Cellulosen und Hydroxyethylcellulosen enthalten zum Beispiel 8 bis 30 Kohlenstoffatome. Die Arylreste sind z. B. aus Phenyl, Benzyl-, Naphthyl-und Anthryl Gruppen gewählt. Beispiele für quaternierte alkylhydroxyethyl-Cellulosen aus C8-C30 Fettsäureketten sind die Produkte Quatrisoft LM 200, LM-X Quatrisoft 529-18-A, Quatrisoft LM-X 529-18B (C12 alkyl) und Quatrisoft LM-X 529 -8 (C18) von der Firma Amerchol verkauft, und die Produkte Crodacel QM, Crodacel QL (C12 alkyl) und Crodacel QS (C18) von der Firma Croda. Beispiele für Polyacrylate mit Aminoseitenketten sind die Polymere 8781-124B oder 9492-103 und Structure Plus von der Firma National Starch.

Unter amphoteren amphiphilen Polymere mit mindestens einer hydrophilen Einheit und mindestens eine Fettsäure-Ketten-Einheit, sind zum Beispiel Methacrylamidopropyltrimethylammonium / Acrylsäure / C10-C30 Alkylmethacrylat-Copolymere zu nennen, wobei der Alkylrest zum Beispiel ein Stearylrest ist.

Die assoziativen Verdickungsmittel in den kosmetischen Zusammensetzungen können beispielsweise in Lösung oder in Dispersion mit einer Konzentration von 1% aktives Material in Wasser und mit einer Viskosität, gemessen mit einem Rheomat RM 180 Rheometer bei 25 ° C, die größere als 0,1 cps und vorzugsweise größer als 0,2 cp, bei einer Scherrate von 200 s⁻¹ eingesetzt werden.

Zusammenfassend sind bevorzugte erfindungsgemäße kosmetische Zusammensetzungen dadurch gekennzeichnet, daß die kationischen amphiphilen Polymere unter den quaternisierten Cellulosederivaten und den Polyacrylaten mit aminierten Seitenketten ausgewählt sind.

Weitere bevorzugte kosmetische Zusammensetzungen sind dadurch gekennzeichnet, daß die amphoteren amphiphilen Polymere, die mindestens eine Fettkette enthalten, unter den Copolymeren von Methacrylamidopropyltrimethylammoniumchlorid/Acrylsäure/Al kyl(C₁₀-₃₀)methacrylat ausgewählt sind.

Erfindungsgemäß bevorzugte kosmetische Zusammensetzungen enthalten das bzw. die Verdickungsmittel in einer Gesamtmenge von 0,001 bis 20 Gew.-%, vorzugsweise von 0,01 bis 15 Gew.-%, weiter bevorzugt von 0,1 bis 10 Gew.-% und insbesondere von 025 bis 5 Gew.%, bezogen auf das Gesamtgewicht der Zusammensetzung,.

Die erfindungsgemäßen Mittel enthalten je nach Anwendungszweck weitere wesentliche Inhaltsstoffe. Reinigende oder pflegende Zusammensetzungen wie beispielsweise Shampoos oder Conditioner enthalten mindestens ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

Erfindungsgemäß bevorzugte Kosmetische Mittel sind dadurch gekennzeichnet, daß sie - bezogen auf ihr Gewicht - 0,5 bis 70 Gew.-%, vorzugsweise 1 bis 60 Gew.-% und insbesondere 5 bis 25

Gew.-% anionische(s) und/oder nichtionische(s) und/oder kationische(s) und/oder amphotere(s) Tensid(e), enthalten.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 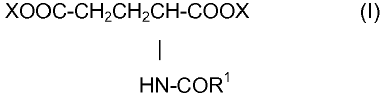 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 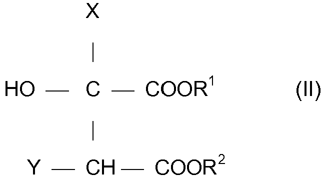 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 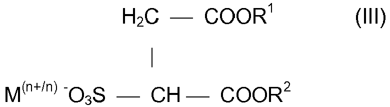 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Weiter bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder Ammoniumsalze des Laurylethersulfates mit einem Ethoxylierungsgrad von 2 bis 4 EO.

Insbesondere bevorzugte erfindungsgemäße Kosmetische Mittel sind dadurch gekennzeichnet, daß sie - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

**H₃C-(CH₂)ₙ-(OCH₂CH₂)ₖ-OSO₃**⁻ **M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, stehen.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin.

Besonders bevorzugte erfindungsgemäße kosmetische Mittel sind dadurch gekennzeichnet, daß sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂-C₁₈-Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Besonders bevorzugte Kosmetische Mittel enthalten als amphotere Tenside Betaine der Formel (Bet-I) 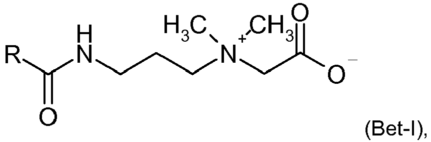 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-I) enthalten.

Zusätzlich zu dem bzw. den Amphotensiden der Formel (Bet-I) oder an deren Stelle können die erfindungsgemäßen Kosmetische Mittel mit besonderem Vorzug als amphotere Tenside Betaine der Formel (Bet-II) 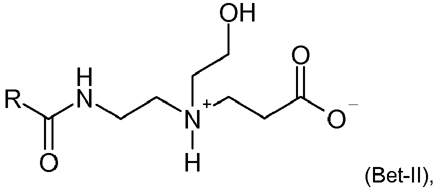 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (Bet-IIa) 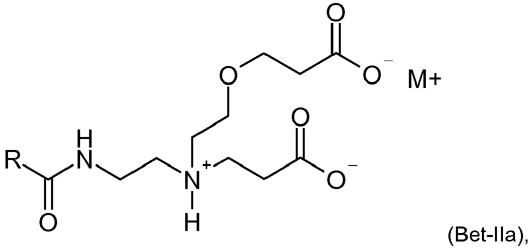 in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (Bet-II) eingesetzt, die ein Gemisch der folgenden Vertreter sind:

H₃C-(CH₂)₇-C(O)-NH-(CH₂)₂NH⁺(CH₂CH₂OH)CH₂CH₂COO⁻

H₃C-(CH₂)₉-C(O)-NH-(CH₂)₂NH⁺(CH₂CH₂OH)CH₂CH₂COO⁻

H₃C-(CH₂)ₙ-C(O)-NH-(CH₂)₂NH⁺(CH₂CH₂OH)CH₂CH₂COO⁻

H₃C-(CH₂)₁₃-C(O)-NH-(CH₂)₂NH⁺(CH₂CH₂OH)CH₂CH₂COO⁻

H₃C-(CH₂)₁₅-C(O)-NH-(CH₂)₂NH⁺(CH₂CH₂OH)CH₂CH₂COO⁻

H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (Bet-II) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-II) enthalten.

Zusammenfassend sind erfindungsgemäße kosmetische Mittel bevorzugt, bei denen der Rest R in den Formeln (Bet-I) und (Bet-II) ausgewählt ist aus H₃C-(CH₂)₇-, H₃C-(CH₂)₉-, H₃C-(CH₂)₁₁-, H₃C-(CH₂)₁₃-, H₃C-(CH₂)₁₅-, H₃C-(CH₂)₇-CH=CH-(CH₂)₇- oder Mischungen aus diesen.

Besonders bevorzugte nichtionische Tenside sind Alkylpolyglycoside. Demnach sind erfindungsgemäße Kosmetische Mittel bevorzugt, die als nichtionische Tenside - bezogen auf ihr Gewicht - 0,1 bis 20 Gew.-% Alkylpolyglycoside der allgemeinen Formel RO-(Z)ₓ enthalten, wobei R für Alkyl, Z für Zucker sowie x für die Anzahl der Zuckereinheiten steht.

Erfindungsgemäß bevorzugt werden Alkylpolyglycoside entsprechend der allgemeinen Formel RO-(Z)ₓ eingesetzt, wobei R für Alkyl, Z für Zucker sowie x für die Anzahl der Zuckereinheiten steht. Besonders bevorzugt sind solche Alkylpolyglycoside, bei denen R
- im wesentlichen aus C₈- und C₁₀-Alkylgruppen,
- im wesentlichen aus C₁₂- und C,₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Weitere Tenside, die - insbesondere in Mischung mit Alkylpolyglycosiden - besonders vorteilhaft in den erfindungsgemäßen Mitteln eingesetzt werden können, sind Glutamate, Asparaginate und Sulfoacetate. Hier sind erfindungsgemäße Kosmetische Mittel bevorzugt, die - bezogen auf ihr Gewicht - 0,1 bis 20 Gew.-% Fettsäureglutamate (Acylglutamate) und/oder Fettsäureasparaginate (Acylasparaginate) und/oder Alkylsulfoacetate (Sulfoessigsäure-alkylester) enthalten.

Acylglutamate lassen sich durch die Formel 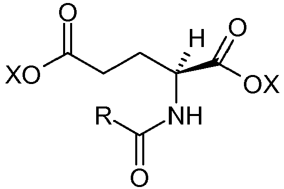 beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

Überraschenderweise wurde gefunden, daß Abmischungen von Alkylglucosiden mit Acylglutamaten eine sehr gute dermatologische Verträglichkeit und ein verbessertes Schaumvermögen sowohl hinsichtlich des Basisschaums als auch der Schaumstabilität in Gegenwart von Wasserhärte aufweisen.

Typische Beispiele für geeignete Acylglutamate sind Aniontenside, die sich von Fettsäuren mit 6 bis 22, vorzugsweise 12 bis 18 Kohlenstoffatomen ableiten, wie beispielsweise C12/14- bzw. C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-glutamat

Die erfindungsgemäßen Mittel können die Alkyl- und/oder Alkenyloligoglucoside und die Acylglutamate im Gewichtsverhältnis 1 : 99 bis 99 : 1, vorzugsweise 10 : 90 bis 90 : 10 und insbesondere 80 : 20 bis 50 : 50 enthalten.

Acylasparaginate lassen sich durch die Formel 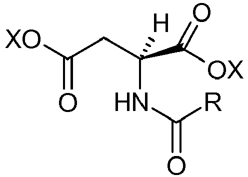 beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

Typische Beispiele für geeignete Acylasparaginate sind Aniontenside, die sich von Fettsäuren mit 6 bis 22, vorzugsweise 12 bis 18 Kohlenstoffatomen ableiten, wie beispielsweise C12/14- bzw. C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-asparaginat

Die erfindungsgemäßen Mittel können die Alkyl- und/oder Alkenyloligoglucoside und die Acylasparaginate ebenfalls im Gewichtsverhältnis 1 : 99 bis 99 : 1, vorzugsweise 10 : 90 bis 90 : 10 und insbesondere 80 : 20 bis 50 : 50 enthalten.

Sulfoacetate (Sulfoessigsäure-ester), sind üblicherweise Salze von Estern der Sulfoessigsäure und lassen sich durch die allgemeine Formel

R-O-C(O)-CH₂-SO₂-OX

beschreiben, in der R für einen linearen oder verzweigten Alkyl- oder Alkenylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

Besonders bevorzugt ist der Einsatz vom Natriumsalz der Sulfoessigsäure mit der INCI-Bezeichnung: Sodium Lauryl Sulfoacetate): H₃C-(CH₂)₁₁-O-CO-CH₂-SO₂-ONa Natriumlaurylsulfoacetat ist ein weißes, freifließendes Pulver, das neutral reagiert, mit gutem Schaumvermögen, Netzvermögen und Dispergiervermögen.

Erfindungsgemäß einsetzbar sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Besonders bevorzugte erfindungsgemäße Haarreinigung sind dadurch gekennzeichnet, daß sie als kationischen Pflegestoff - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Tensid(e) aus der Gruppe der quartären Ammoniumverbindungen und/oder der Esterquats und/oder der Amidoamine enthalten, wobei bevorzugte kationische(s) Tensid(e) ausgewählt ist/sind aus
- Alkyltrimethylammoniumchloriden mit vorzugsweise 10 bis 18 C-Atomen im Alkylrest und/oder
- Dialkyldimethylammoniumchloride mit vorzugsweise 10 bis 18 C-Atomen im Alkylrest und/oder
- Trialkylmethylammoniumchloride mit vorzugsweise 10 bis 18 C-Atomen im Alkylrest und/oder
- Cetyltrimethylammoniumchlorid und/oder
- Stearyltrimethylammoniumchlorid und/oder
- Distearyldimethylammoniumchlorid und/oder
- Lauryldimethylammoniumchlorid und/oder
- Lauryldimethylbenzylammoniumchlorid und/oder
- Tricetylmethylammoniumchlorid
- Quaternium-27 und/oder
- Quaternium-83 und/oder
- N-Methyl-N(2-hydroxyethyl)-N,N-(ditalgacyloxyethyl)ammonium-methosulfat und/oder
- N-Methyl-N(2-hydroxyethyl)-N,N-(distearoyloxyethyl)ammonium-methosulfat und/oder
- N,N-Dimethyl-N,N-distearoyloxyethyl-ammoniumchlorid und/oder
- N,N-Di-(2-hydroxyethyl)-N,N-(fettsäureesterethyl)-ammoniumchlorid.

Die Pflegeffekte der erfindungsgemäßen Mittel lassen sich noch weiter verstärken, indem bestimmte Pflegestoffe eingesetzt werden. Vorzugsweise werden diese aus bestimmten Gruppen an sich bekannter Pflegestoffe ausgewählt, da diese Pflegestoffe formulierungstechnisch und vom Pflegeffekt hervorragend mit den erfindungsgemäß eingesetzten Silikonen harmonieren. Erfindungsgemäß bevorzugte Kosmetische Mittel sind dadurch gekennzeichnet, daß sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i. L-Carnitin und/oder seiner Salze;
ii. Panthenol und/oder Panthothensäure;
iii. der 2-Furanone und/oder deren Derivate, insbesondere Pantolacton;
iv. Taurin und/oder seiner Salze;
v. Niacinamid;
vi. Ubichinon
vii. Ectoin;
viii. Allantoin.

In erfindungsgemäßen Haarbehandlungsmitteln dieser Ausführungsform werden die erfindungsgemäß eingesetzten Silikone mit mindestens einem Pflegestoff kombiniert, der ausgewählt ist aus L-Carnitin und/oder seinen Salzen, Panthenol und/oder Panthothensäure, 2-Furanonen und/oder deren Derivaten, insbesondere Pantolacton, Taurin und/oder seinen Salzen, Niacinamid, Ubichinonen, Ectoin, Allantoin. Diese Pflegestoffe werden nachstehend beschrieben L-Carnitin (IUPAC-Name(R)-(3-Carboxy-2-hydroxypropyl)- *N,N*,*N*-trimethylammoniumhydroxid), ist eine natürlich vorkommende, vitaminähnliche Substanz. Es spielt eine essentielle Rolle im Energiestoffwechsel menschlicher, tierischer und pflanzlicher Zellen. L-Carnitin kann über verschiedene Wege im industriellen Maßstab gewonnen werden. Beispielsweise über einen, die körpereigene Biosynthese imitierenden, biotechnologischen Prozess: In großen Fermentationsbehältern wird dabei die Vorstufe von L-Carnitin (γ-Butyrobetain) mit Hilfe von gramnegativen Bakterien (Rhizobien) in L-Carnitin umgesetzt.

Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Erfindungsgemäße bevorzugte Kosmetische Mittel sind dadurch gekennzeichnet, daß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5).

Erfindungsgemäße bevorzugte Kosmetische Mittel sind dadurch gekennzeichnet, daß sie - bezogen auf sein Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-N-(3-hydroxypropyl)-3,3-dimethyl-butyramid) enthalten.

Ein weiterer, bevorzugter einsetzbarer Pflegestoff, der aktivierende Eigenschaften besitzt, ist das Taurin. Erfindungsgemäß bevorzugte Kosmetische Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1-5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Kosmetische Mittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflegestoff besitzen. Besonders bevorzugte erfindungsgemäße Kosmetische Mittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 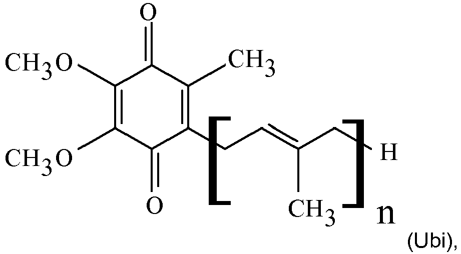 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 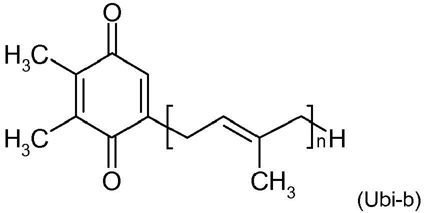 in der n für Werte von 1 bis 20, vorzugsweise von 2 bis 15 und insbesondere für 5, 6, 7, 8, 9, 10 steht, wobei besonders bevorzugt Mittel Plastochinon PQ-9 enthalten.

Als weiteren Pflege-Enhacer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Kosmetische Mittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Ein weiterer Pflegestoff ist Allantoin. Das Allantoin *(5-Ureidohydantoin, N*-(2,5-Dioxo-4-imidazolidinyl)-harnstoff) ist bei verschiedenen Tierarten, vor allem bei Säugetieren, neben der Harnsäure das Endprodukt des Abbaus von Nukleinsäuren, speziell von Purinbasen.

Allantoin wird in der Kosmetik in Hautcremes, Sonnenschutzmitteln, Rasierwässern, in Zahncreme und in Mitteln gegen übermäßige Schweißabsonderung (Hyperhidrose) und Hautirritationen eingesetzt. Es bewirkt die Beschleunigung des Zellaufbaus, der Zellbildung oder der Zellregeneration und beruhigt die Haut. Auch die Heilung schwer heilender Wunden wird unterstützt, jedoch besitzt Allantoin keine antiseptischen Eigenschaften.

Erfindungsgemäße besonders bevorzugte Kosmetische Mittel enthalten - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin).

Zur Verbesserung der Elastizität und Festigung der inneren Struktur der mit erfindungsgemäßen Mitteln behandelter Haare können die erfindungsgemäßen Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, daß die mit entsprechenden Mitteln behandelten Haare unter anderem höhere Meßwerte bei der Differenzthermoanalyse und verbesserte Naß- und Trockenkämmbarkeiten zeigen.

Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Mittel enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Kosmetische Mittel sind dadurch gekennzeichnet, daß sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 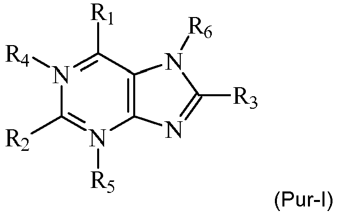 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Kosmetische Mittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflegestoff in den erfindungsgemäßen Mitteln. Hier sind erfindungsgemäße Kosmetische Mittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Kosmetische Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methylguanidino-essigsäure (Creatin).

Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zur Bekämpfung der hormonellen Einflüße auf die Haarfollikel kann zur äußeren Anwendung Spironolactone in Form von Haarwasser und in Kombination mit Minoxidil angewandt werden.

Spironolactone wirkt als Androgen-Rezeptor-Blocker, dh. die Bindung von DHT an die Haarfollikel wird verhindert.

Zusammenfassend sind erfindungsgemäße Kosmetische Mittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.

Zusammenfassend sind erfindungsgemäße Kosmetische Mittel bevorzugt, die zusätzlich - bezogen auf ihr Gewicht - 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder Zink-Pyrithion und/oder Selensulfid und/oder Climbazol und/oder Salicylsäure oder Fumarsäure enthalten.

Zusätzlich zu den Pflegestoffen können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, *S*-Methyl-L-methionin, *S*-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Kosmetische Mittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Kosmetische Mittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus

Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose, Disacchariden, insbesondere Saccharose und/oder Maltose und/oder Lactose und/oder Trehalose und/oder Cellobiose und/oder Gentiobiose und/oder Isomaltose.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte erfindungsgemäße Mittel (eine) Aminosäure(n). Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3',4'-Dihydroxy-L-phenylalanin (L-Dopa), 5'-Hydroxy-L-tryptophan, L-Homocystein, *S*-Methyl-L-methionin, *S*-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Die erfindungsgemäß eingesetzten Silikone können selbstverständlich mit weiteren herkömmlichen Silikonen gemeinsam eingesetzt werden.

Erfindungsgemäße bevorzugte Mittel sind dadurch gekennzeichnet, daß sie mindestens ein weiteres Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die weiteren Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Mittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Es werden im Rahmen der vorliegenden Erfindung als Silicon der Formel Si-I vorzugsweise die Verbindungen: (CH₃)₃Si-O-Si(CH₃)₃, (CH₃)₃Si-O-(CH₃)₂Si-O-Si(CH₃)₃, (CH₃)₃Si-[O-(CH₃)₂Si]₂-O-Si(CH₃)₃, (CH₃)₃Si-[O-(CH₃)₂Si]₃-O-Si(CH₃)₃, (CH₃)₃Si-[O-(CH₃)₂Si]₄-O-Si(CH₃)₃, (CH₃)₃Si-[O-(CH₃)₂Si]₅-O-Si(CH₃)₃, (CH₃)₃Si-[O-(CH₃)₂Si]₆-O-Si(CH₃)₃, (CH₃)₃Si-[O-(CH₃)₂Si]₇-O-Si(CH₃)₃, (CH₃)₃Si-[O-(CH₃)₂Si]₈-O-Si(CH₃)₃, (CH₃)₃Si-[O-(CH₃)₂Si]₉-O-Si(CH₃)₃, (CH₃)₃Si-[O-(CH₃)₂Si]₁₀-O-Si(CH₃)₃, (CH₃)₃Si-[O-(CH₃)₂Si]₁₁-O-Si(CH₃)₃, (CH₃)₃Si-[O-(CH₃)₂Si]₁₂-O-Si(CH₃)₃, (CH₃)₃Si-[O-(CH₃)₂Si]₁₃-O-Si(CH₃)₃, (CH₃)₃Si-[O-(CH₃)₂Si]₁₄-O-Si(CH₃)₃, (CH₃)₃Si-[O-(CH₃)₂Si]₁₅-O-Si(CH₃)₃, (CH₃)₃Si-[O-(CH₃)₂Si]₁₆-O-Si(CH₃)₃, (CH₃)₃Si-[O-(CH₃)₂Si]₁₇-O-Si(CH₃)₃, (CH₃)₃Si-[O-(CH₃)₂Si]₁₈-O-Si(CH₃)₃, (CH₃)₃Si-[O-(CH₃)₂Si]₁₉-O-Si(CH₃)₃, (CH₃)₃Si-[O-(CH₃)₂Si]₂₀-O-Si(CH₃)₃ eingesetzt, wobei (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind. Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist-N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃,-CH₂CH₂CH₂CH₃, -O-CH₂CH(CH₃)₂, -CH₂CH(CH₃)₂, -O-CH(CH₃)CH₂CH₃,-CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie mindestens ein aminofunktionelles Silikon der Formel (Si-IIa) 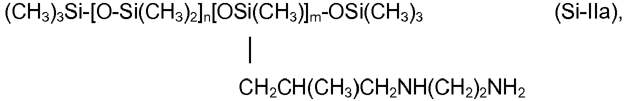 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet. Besonders bevorzugt sind auch erfindungsgemäße Mittel, die ein aminofunktionelles Silikon der Formel (Si-IIb) 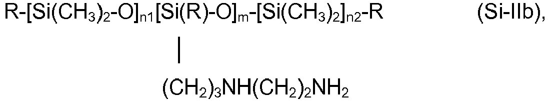 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Mittel sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III

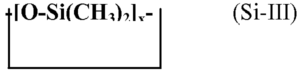

enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz. Erfindungsgemäß ebenfalls bevorzugte Mittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H,-Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel sind dadurch gekennzeichnet, daß sie mindestens ein wasserlösliches Silikon enthalten.

Als weiteren Inhaltsstoff können die erfindungsgemäßen Mittel mindestens ein Proteolipid der Formel (P-I) enthalten

**R'-X-R"** (P-I),

in der
- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für -C(O)O- oder -N⁺(R^{III}₂)R^{IV}- oder -N(R^{III})R^{IV}- oder -C(O)-N(R^{V})R^{VI}- steht,
- R^{III} -(CH₂)ₓ-CH₃ mit x = 0 - 22 bedeutet und
- R^{IV} -CH₂-CH(OH)-CH₂- oder -(CH₂)ₓ- mit x = 0 - 22 bedeutet;
- R^{V} und R^{V} unabhängig voneinander für -H oder -(CH₂)ₓ-CH₃ mit x = 0 - 22 stehen;
mit der Maßgabe, daß R" für Keratin oder ein Keratinhydrolysat steht, wenn X für -C(O)O- steht. Vorzugsweise werden die Proteolipide innerhalb bestimmter Mengen in den erfindungsgemäßen Mitteln eingesetzt. Bevorzugte erfindungsgemäße Kosmetische Mittel enthalten - bezogen auf ihr Gewicht - 0,01 bis 10 Gew.-%, vorzugsweise 0,02 bis 5 Gew.-%, besonders bevorzugt 0,05 bis 2,5 Gew.-%, weiter bevorzugt 0,1 bis 1 Gew.-% und insbesondere 0,15 bis 0,5 Gew.-% Proteolipid(e). Der Rest R" in Formel (P-I) steht für ein Peptid oder ein Protein oder ein Proteinhydrolysat. Wenn X = -C(O)O-, wird R" aus der Gruppe Keratin oder Keratinhydrolysat gewählt.

Bevorzugte Reste R" sind Oligopetide, die mindestens eine Aminosäuresequenz Glu-Glu-Glu aufweisen, wobei die Amino-Gruppe frei oder protoniert und die Carboxy-Gruppen frei oder deprotoniert vorliegen können.

In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie dargestellt (in der vorstehenden Formel 3) oder aber, daß die Aminosäuresequenz in einem Oligopeptid vorliegt, das noch weitere Anminosäuren umfaßt - je nachdem, wo die weitere(n) Aminosäure(n) gebunden ist/sind, sind die eingeklammerten Bestandteile der o.g. Formel durch den/die weiteren Aminosäurerest(e) ersetzt. Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen

In erfindungsgemäß bevorzugten Haarbehandlungsmitteln der vorstehend beschriebenen Ausführungsform umfaßt das Oligopeptid (= der Rest R") 5 bis 15 Aminosäuren, vorzugsweise 6 bis 13 Aminosäuren, besonders bevorzugt 7 bis 12 Aminosäuren und insbesondere 8, 9 oder 10 Aminosäuren.

Je nachdem, ob weitere Aminosäuren an die Sequenz Glu-Glu-Glu gebunden sind und je nach Art dieser Aminosäuren sowie in Abhängigkeit von der Auswahl der Reste R' und ggf. R^{III} und R^{IV} kann die Molmasse des in den erfindungsgemäßen Mitteln enthaltenen Proteolipids variieren. Erfindungsgemäß bevorzugte Kosmetische Mittel sind dadurch gekennzeichnet, daß das Proteolipid eine Molmasse von 1000 bis 30000 Da, vorzugsweise von 1250 bis 25000 Da, besonders bevorzugt von 1500 bis 20000 Da und insbesondere von 2000 bis 15000 Da aufweist. Als Rest R" werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind.

So ist es bevorzugt, wenn der Rest R" der in den erfindungsgemäßen Mitteln eingesetzten Proteolipide kein Methionin enthält. Weiter bevorzugt ist es, wenn der Rest R" der in den erfindungsgemäßen Mitteln eingesetzten Proteolipide kein Cystein und/oder Cystin enthält.

Weiter bevorzugt ist es, wenn der Rest R" der in den erfindungsgemäßen Mitteln eingesetzten Proteolipide keine Asparaginsäure und/oder Asparagin enthält. Weiter bevorzugt ist es, wenn der Rest R" der in den erfindungsgemäßen Mitteln eingesetzten Proteolipide kein Serin und/oder Threonin enthält.

Demgegenüber ist es bevorzugt, wenn der Rest R" der in den erfindungsgemäßen Mitteln eingesetzten Proteolipide Tyrosin enthält. Weiter bevorzugt ist es, wenn der Rest R" der in den erfindungsgemäßen Mitteln eingesetzten Proteolipide Leucin enthält. Weiter bevorzugt ist es, wenn der Rest R" der in den erfindungsgemäßen Mitteln eingesetzten Proteolipide Isoleucin enthält. Weiter bevorzugt ist es, wenn der Rest R" der in den erfindungsgemäßen Mitteln eingesetzten Proteolipide Arginin enthält. Weiter bevorzugt ist es, wenn der Rest R" der in den erfindungsgemäßen Mitteln eingesetzten Proteolipide Valin enthält.

Als Rest R" besonders bevorzugte Oligopeptide bzw in den bevorzugten Oligopeptiden enthaltene Aminosäuresequenzen werden nachstehend beschrieben:
Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Glu-Glu-Glu-Ile aufweist, wobei die Amino-Gruppe frei oder protoniert und die Carboxy-Gruppen frei oder deprotoniert vorliegen können.

Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in den Proteolipiden der Formel (I) als Rest R" enthaltene Oligopeptid mindestens eine Aminosäuresequenz Tyr-Glu-Glu-Glu-Ile aufweist, wobei die Amino-Gruppe frei oder protoniert und die Carboxy-Gruppen frei oder deprotoniert vorliegen können. Weiter bevorzugte Oligopeptide enthalten zusätzlich Arginin, das vorzugsweise an Isoleucin gebunden vorliegt. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg aufweist, wobei die Amino-Gruppen frei oder protoniert und die Carboxy-Gruppen frei oder deprotoniert vorliegen können.

Noch weiter bevorzugte Oligopeptide enthalten zusätzlich Valin, das vorzugsweise an das Arginin gebunden vorliegt. Erfindungsgemäß weiter bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val aufweist, wobei die Amino-Gruppen frei oder protoniert und die Carboxy-Gruppen frei oder deprotoniert vorliegen können. Noch weiter bevorzugte Oligopeptide enthalten zusätzlich Leucin, das vorzugsweise an Valin gebunden vorliegt. Erfindungsgemäß weiter bevorzugte Kosmetische Mittel sind dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val-Leu aufweist, wobei die Amino-Gruppen frei oder protoniert und die Carboxy-Gruppen frei oder deprotoniert vorliegen können. Insbesondere bevorzugte Oligopeptide enthalten zusätzlich Leucin, das vorzugsweise an das Tyrosin gebunden vorliegt. Erfindungsgemäß weiter bevorzugte Kosmetische Mittel sind dadurch gekennzeichnet, daß das in den Proteolipiden der Formel (I) als Rest R" enthaltene Oligopeptid mindestens eine Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können.

Zusammenfassend sind insbesondere erfindungsgemäße Kosmetische Mittel bevorzugte, die mindestens ein Proteolipd der Formel (I) enthalten, in der R" mindestens eine Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können.

Wie bereits erwähnt, wird R" aus der Gruppe Keratin oder Keratinhydrolysat gewählt, wenn X = -C(O)O- gilt.

In allen anderen Fällen kann der Rest R" in Formel (P-I) für ein Peptid oder ein Protein oder ein Proteinhydrolysat stehen, wobei Proteinhydrolysate bevorzugt sind. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Vorzugsweise wird unabhängig von der Wahl des X in Formel (P-I) der Rest R" aus Keratin oder keratinhydrolxysaten ausgewählt. Bevorzugte erfindungsgemäße Kosmetische Mittel sind dadurch gekennzeichnet, daß sie mindestens ein Proteolipid der Formel (P-I) enthalten, in der R" für Keratin oder ein Keratinhydrolysat steht.

Insbesondere sind erfindungsgemäße Kosmetische Mittel bevorzugt, die mindestens ein Proteolipd der Formel (P-I) enthalten, in der R^{III} -CH₃ bedeutet und R^{IV} für -(CH₂)ₓ- mit x = 0, 1, 2, 3, 4, 5, 6, 7, 8 steht.

Weiter sind besonders bevorzugte erfindungsgemäße Kosmetische Mittel dadurch gekennzeichnet, daß sie mindestens ein Proteolipd der Formel (I) enthalten, in der X für -N⁺(CH₃)₂-CH₂-CH(OH)-CH₂- und R' für -(CH₂)₁₇-CH₃ steht.

Ebenfalls weiter bevorzugte erfindungsgemäße Kosmetische Mittel dadurch gekennzeichnet, daß sie mindestens ein Proteolipd der Formel (P-I) enthalten, in der X für -C(O)-O- und R' für -(CH₂)₁₇-CH₃ steht.

Es hat sich als vorteilhaft erwiesen, zusätzlich zu den Proteolipiden Proteinhydrolysate einzusetzen. Diese verstärken die Wirkung der Proteolipide und werden ihrerseits in ihren Effekten verstärkt. Die Proteinhydrolysate wurden weiter oben als Rest R" detailliert beschrieben. Zusammenfassend sind erfindungsgemäße Kosmetische Mittel bevorzugt, die zusätzlich - bezogen auf ihr Gewicht - 0,01 bis 10 Gew.-%, vorzugsweise 0,05 bis 7 Gew.-%, besonders bevorzugt 0,1 bis 5 Gew.-%, weiter bevorzugt 0,25 bis 2,5 Gew.-% und insbesondere 0,5 bis 2,0 Gew.-% Proteinhydrolysat(e), vorzugsweise Keratinhydrolysat(e) enthalten.

Aus ästhetischen Gründen werden "klare" Produkte von Verbrauchern oft bevorzugt. Erfindungsgemäß bevorzugte Kosmetische Mittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit. Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Ein weiterer Gegenstand der vorliegenden Erfindung ist ein Verfahren zur Behandlung keratinischer Fasern, bei dem ein erfindungsgemäßes Haarbehandlungsmittel auf die keratinischen Fasern aufgetragen wird und nach einer Einwirkzeit von wenigen Sekunden bis hin zu 45 Minuten wieder ausgespült wird.

Bezüglich bevorzugter Ausführungsformen der erfindungsgemäßen Verfahren gilt mutatis mutandis das zu den erfindungsgemäßen Mitteln Gesagte.

Ein weiterer Gegenstand der vorliegenden Erfindung ist die Verwendung von erfindungsgemäßen Haarbehandlungsmitteln
- zum Konditionieren von Keratinsubstanzen und/oder
- um die Lockerheit, die Weichheit, den Glanz und/oder die Kämmbarkeit zu verbessern und das Frisieren von Keratinsubstanzen zu erleichtern und/oder
- um die Remanenz der Konditionierwirkung bei der Haarwäsche zu verbessern und/oder
- zur Verbesserung der Nass- und Trockenkämmbarkeiten und/oder
- zur Verbesserung des Glanzes und/oder
- zur Verbesserung des Feuchtehaushaltes keratinischer Fasern und/oder
- zum Schutz der keratinischen Fasern vor oxidativen Schädigungen und/oder
- zum Verhindern des Nachfettens keratinischer Fasern und/oder
- zur Erhöhung der Waschbeständigkeit gefärbter keratinischer Fasern.

Auch bezüglich bevorzugter Ausführungsformen der erfindungsgemäßen Verwendungen gilt mutatis mutandis das zu den erfindungsgemäßen Mitteln Gesagte.

## Patentansprüche

1. Kosmetische Zusammensetzung, enthaltend in einem kosmetisch akzeptablen Medium
(a) mindestens ein Verdickungsmittel, ausgewählt aus assoziativen Verdickungsmitteln
(b) mindestens ein hydroxy-terminiertes Organopolysiloxan der allgemeinen Formel (I), 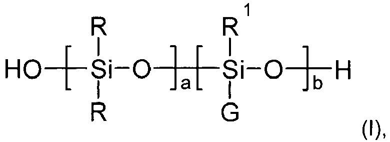 wobei
- R einen einwertigen unsubstituierten oder halogensubstituierten Kohlenwasserstoffrest mit 1 bis 20 Kohlenstoffatomen,
- R¹ einen einwertigen unsubstituierten oder halogensubstituierten Kohlenwasserstoffrest mit 1 bis 20 Kohlenstoffatomen, -OR⁴ oder -OH,
- R⁴ einen Alkylrest mit 1 bis 6 Kohlenstoffatomen,
- G eine Gruppe der allgemeinen Formel (II) 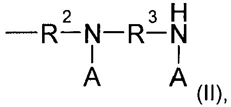 wobei
- R², R³ unabhängig voneinander einen zweiwertigen Kohlenwasserstoffrest mit 1 bis 6 Kohlenstoffatomen, wobei nicht benachbarte -CH₂-Einheiten ersetzt sein können durch Einheiten, die ausgewählt werden aus -C(=O)-, -O-, und -S-,
- A R⁵-C(=O)-,
- R⁵ einen Alkylrest mit 1 bis 20 Kohlenstoffatomen,
- a ganzzahlige Werte von 100 bis 1500 und
- b ganzzahlige Werte von mindestens 1 bedeuten.

2. Kosmetische Zusammensetzung nach Anspruch 1, **dadurch gekennzeichnet, daß** sie mindestens ein hydroxy-terminiertes Organopolysiloxan der allgemeinen Formel (I) enthält, in der R ausgewählt ist aus Methyl-, Ethyl-, n-Propyl, Isopropyl-, n-Butyl-, Isobutyl-, sec-Butyl-, tert-Butyl, n-Pentyl, n-Hexyl-Resten, wobei R äußerst bevorzugt für Methyl steht.

3. Kosmetische Zusammensetzung nach einem der Ansprüche 1 oder 2, **dadurch gekennzeichnet, daß** sie mindestens ein hydroxy-terminiertes Organopolysiloxan der allgemeinen Formel (I) enthält, in der die Reste R², R³ ausgewählt sind aus Ethylen-, n-Propylen-, iso-Butylen- oder n-Butylenresten.

4. Kosmetische Zusammensetzung nach einem der Ansprüche 1 bis 3, **dadurch gekennzeichnet, daß** sie mindestens ein hydroxy-terminiertes Organopolysiloxan der allgemeinen Formel (I) enthält, in der R⁵ ausgewählt ist aus Methyl-, Ethyl-, n-Propyl, Isopropyl-, n-Butyl-, Isobutyl-, sec-Butyl-, tert-Butyl, n-Pentyl, n-Hexyl-Resten, wobei R⁵ äußerst bevorzugt für Methyl oder Ethyl steht.

5. Kosmetische Zusammensetzung nach einem der Ansprüche 1 bis 4, **dadurch gekennzeichnet, daß** sie - bezogen auf ihr Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% hydroxy-terminierte(s) Organopolysiloxan(e) der allgemeinen Formel (I) enthält.

6. Kosmetische Zusammensetzung nach einem der Ansprüche 1 bis 5, **dadurch gekennzeichnet, daß** sie - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-w-hydroxypolyglycolether (INCI-Bezeichnung: Trideceth-10) oder deren Mischungen enthält.

7. Kosmetische Zusammensetzung nach einem der Ansprüche 1 bis 6, **dadurch gekennzeichnet, daß** die gewichtmittlere Molmasse des hydroxy-terminierten Organopolysiloxans der allgemeinen Formel (I), im Bereich von 2.000 bis 1.000.000 gmol⁻¹, vorzugsweise im Bereich von 5.000 bis 200.000 gmol⁻¹ liegt.

8. Kosmetische Zusammensetzung nach einem der Ansprüche 1 bis 7, **dadurch gekennzeichnet, daß** das hydroxy-terminierte Organopolysiloxan der allgemeinen Formel (I) in Form einer Öl-in-Wasser-Emulsion vorliegt, in der die zahlenmittlere Größe der Siliconpartikel in der Emulsion im Bereich von 3 bis 500 nm, vorzugsweise im Bereich von 5 bis 60 nm liegt.

9. Kosmetische Zusammensetzung nach einem der Ansprüche 1 bis 8, **dadurch gekennzeichnet, daß** das assoziative Verdickungsmittel ein assoziatives Polymer ist, das ausgewählt ist unter
(i.) nichtionischen amphiphilen Polymeren, die mindestens ein Fettkette und mindestens eine hydrophile Einheit enthalten;
(ii.) anionischen amphiphilen Polymeren, die mindestens eine hydrophile Einheit und mindestens eine Einheit mit Fettkette enthalten;
(iii.) kationischen amphiphilen Polymeren, die mindestens eine hydrophile Einheit und mindestens eine Einheit mit Fettkette enthalten;
(iv.) amphoteren amphiphilen Polymeren, die mindestens eine hydrophile Einheit und mindestens eine Einheit mit Fettkette enthalten,
wobei die Fettketten 10 bis 30 Kohlenstoffatome aufweisen.

10. Kosmetische Zusammensetzung nach Ansprüche 1 bis 8, **dadurch gekennzeichnet, daß** die nichtionischen amphiphilen Polymere, die mindestens eine Fettkette und mindestens eine hydrophile Einheit enthalten, vorzugsweise ausgewählt sind unter
(1) Cellulosen, die mit Gruppen modifiziert sind, die mindestens eine Fettkette aufweisen;
(2) Hydroxypropylguarverbindungen, die mit Gruppen modifiziert sind, die mindestens eine Fettkette aufweisen;
(3) Urethanpolyether, die mindestens eine Fettkette enthalten, wie Alkyl- oder Alkenylgruppen mit 10 bis 30 Kohlenstoffatomen;
(4) Copolymeren von Vinylpyrrolidon und hydrophoben Monomeren mit Fettkette;
(5) Copolymeren von Alkyl(C₁₋₆)methacrylaten oder Alkyl(C₁₋₆)-acrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten.

11. Kosmetische Zusammensetzung nach einem der Ansprüche 1 bis 8, **dadurch gekennzeichnet, daß** die anionischen amphiphilen Polymere, die mindestens eine hydrophile Einheit und mindestens eine Einheit mit Fettkette enthalten, unter den Verbindungen, die mindestens eine Allylethereinheit mit Fettkette und mindestens eine hydrophile Einheit enthalten, die aus einem ethylenisch ungesättigten, anionischen Monomer aufgebaut ist, Verbindungen, die mindestens eine hydrophile Einheit vom Typ einer olefinisch ungesättigten Carbonsäure und mindestens eine hydrophobe Einheit ausschließlich vom Typ eines Alkyl(C₁₋₃₀)esters einer ungesättigten Carbonsäure enthalten, und Copolymeren Methacrylsäure/ Methylacrylat/ Dimethyl-meta-isopropenyl-benzylisocyanat eines ethoxylierten Alkohols ausgewählt sind.

12. Kosmetische Zusammensetzung nach einem der Ansprüche 1 bis 8, **dadurch gekennzeichnet, daß** die kationischen amphiphilen Polymere unter den quaternisierten Cellulosederivaten und den Polyacrylaten mit aminierten Seitenketten ausgewählt sind.

13. Kosmetische Zusammensetzung nach einem der Ansprüche 1 bis 8, **dadurch gekennzeichnet, daß** die amphoteren amphiphilen Polymere, die mindestens eine Fettkette enthalten, unter den Copolymeren von Methacrylamidopropyltrimethylammoniumchlorid/Acrylsäure/Alkyl(C₁₀₋₃₀)methacrylat ausgewählt sind.

14. Kosmetische Zusammensetzung nach einem der Ansprüche 1 bis 13 **dadurch gekennzeichnet, daß** sie das bzw. die Verdickungsmittel in einer Gesamtmenge von 0,001 bis 20 Gew.-%, vorzugsweise von 0,01 bis 15 Gew.-%, weiter bevorzugt von 0,1 bis 10 Gew.-% und insbesondere von 0.25 bis 5 Gew.-%, bezogen auf das Gesamtgewicht der Zusammensetzung, enthält.

15. Verfahren zur Behandlung keratinischer Fasern **dadurch gekennzeichnet, dass** ein Haarbehandlungsmittel gemäß einem der Ansprüche 1 bis 14 auf die keratinischen Fasern aufgetragen wird und nach einer Einwirkzeit von wenigen Sekunden bis hin zu 45 Minuten wieder ausgespült wird.

16. Verwendung einer Zusammensetzung gemäß einem der Ansprüche 1 bis 15
- zum Konditionieren von Keratinsubstanzen und/oder
- um die Lockerheit, die Weichheit, den Glanz und/oder die Kämmbarkeit zu verbessern und das Frisieren von Keratinsubstanzen zu erleichtern und/oder
- um die Remanenz der Konditionierwirkung bei der Haarwäsche zu verbessern und/oder
- zur Verbesserung der Nass- und Trockenkämmbärkeiten und/oder
- zur Verbesserung des Glanzes und/oder
- zur Verbesserung des Feuchtehaushaltes keratinischer Fasern und/oder
- zum Schutz der keratinischen Fasern vor oxidativen Schädigungen und/oder
- zum Verhindern des Nachfettens keratinischer Fasern und/oder
- zur Erhöhung der Waschbeständigkeit gefärbter keratinischer Fasern.

## Claims

1. A cosmetic composition containing, in a cosmetically acceptable medium,
(a) at least one thickener selected from associative thickeners,
(b) at least one hydroxy-terminated organopolysiloxane of general formula (I), 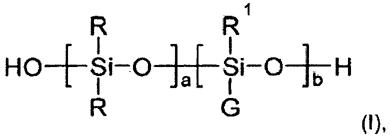 where
- R denotes a monovalent unsubstituted or halogen-substituted hydrocarbon functional group having 1 to 20 carbon atoms,
- R¹ denotes a monovalent unsubstituted or halogen-substituted hydrocarbon functional group having 1 to 20 carbon atoms, -OR⁴ or -OH,
- R⁴ denotes an alkyl functional group having 1 to 6 carbon atoms,
- G denotes a group of general formula (II) 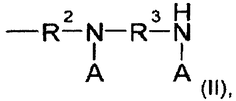
where
- R², R³ independently of one another denote a divalent hydrocarbon functional group having 1 to 6 carbon atoms, wherein non-adjacent -CH₂ units may be replaced by units that are selected from -C(=O)-, -O-, and -S-,
- A denotes R⁵-C(=O)-,
- R⁵ denotes an alkyl functional group having 1 to 20 carbon atoms,
- a denotes integer values of from 100 to 1500 and
- b denotes integer values of at least 1.

2. The cosmetic composition according to claim 1, **characterized in that** it contains at least one hydroxy-terminated organopolysiloxane of general formula (I) in which R is selected from methyl, ethyl, n-propyl, isopropyl, n-butyl, isobutyl, sec-butyl, tert-butyl, n-pentyl and n-hexyl functional groups, R extremely preferably representing methyl.

3. The cosmetic composition according to one of claims 1 or 2, **characterized in that** it contains at least one hydroxy-terminated organopolysiloxane of general formula (I) in which the functional groups R², R³ are selected from ethylene, n-propylene, iso-butylene or n-butylene functional groups.

4. The cosmetic composition according to one of claims 1 to 3, **characterized in that** it contains at least one hydroxy-terminated organopolysiloxane of general formula (I) in which R⁵ is selected from methyl, ethyl, n-propyl, isopropyl, n-butyl, isobutyl, sec-butyl, tert-butyl, n-pentyl and n-hexyl functional groups, R⁵ extremely preferably representing methyl or ethyl.

5. The cosmetic composition according to one of claims 1 to 4, **characterized in that** it contains, based on its weight, from 0.00001 to 10 wt.%, preferably 0.0001 to 7.5 wt.%, particularly preferably 0.001 to 5 wt.%, more preferably 0.01 to 3 wt.% and in particular 0.1 to 1 wt.% hydroxy-terminated organopolysiloxane(s) of general formula (I).

6. The cosmetic composition according to one of claims 1 to 5, **characterized in that** it contains, based on its weight, from 0.00001 to 5 wt.%, preferably 0.0001 to 3.5 wt.%, particularly preferably 0.001 to 2 wt.%, more-preferably 0.01 to 1 wt.% and in particular 0.1 to 0.5 wt.% branched ethoxylated tridecanol (INCI name: Trideceth 5) or α-iso-tridecyl-ω-hydroxy polyglycol ether (INCI name: Trideceth 10) or mixtures thereof.

7. The cosmetic composition according to one of claims 1 to 6, **characterized in that** the weight-average molar mass of the hydroxy-terminated organopolysiloxane of general formula (I) is in the range of from 2,000 to 1,000,000 gmol⁻¹, preferably in the range of from 5,000 to 200,000 gmol⁻¹.

8. The cosmetic composition according to one of claims 1 to 7, **characterized in that** the hydroxy-terminated organopolysiloxane of general formula (I) is in the form of an oil-in-water emulsion in which the number-average size of the silicone particles in the emulsion is in the range of from 3 to 500 nm, preferably in the range of from 5 to 60 nm.

9. The cosmetic composition according to one of claims 1 to 8, **characterized in that** the associative thickener is an associative polymer selected from the group consisting of
(i) non-ionic amphiphilic polymers containing at least one fatty chain and at least one hydrophilic unit;
(ii) anionic amphiphilic polymers containing at least one hydrophilic unit and at least one unit having a fatty chain;
(iii) cationic amphiphilic polymers containing at least one hydrophilic unit and at least one unit having a fatty chain;
(iv) amphoteric amphiphilic polymers containing at least one hydrophilic unit and at least one unit having a fatty chain,
the fatty chains having 10 to 30 carbon atoms.

10. The cosmetic composition according to one of claims 1 to 8, **characterized in that** the non-ionic amphiphilic polymers containing at least one fatty chain and at least one hydrophilic unit are preferably selected from the group consisting of
(1) celluloses modified by groups having at least one fatty chain;
(2) hydroxypropyl guar compounds modified by groups having at least one fatty chain;
(3) urethane polyethers containing at least one fatty chain, such as alkyl or alkenyl groups having 10 to 30 carbon atoms;
(4) copolymers of vinylpyrrolidone and hydrophobic monomers having a fatty chain;
(5) copolymers of alkyl(C₁₋₆)methacrylates or alkyl(C₁₋₆) acrylates and amphiphilic monomers containing at least one fatty chain;
(6) copolymers of hydrophilic methacrylates or acrylates and hydrophobic monomers containing at least one fatty chain.

11. The cosmetic composition according to one of claims 1 to 8, **characterized in that** the anionic amphiphilic polymers containing at least one hydrophilic unit and at least one unit having a fatty chain are selected from the compounds containing at least one allyl ether unit having a fatty chain and at least one hydrophilic unit that is composed of an ethylenically unsaturated anionic monomer, and compounds containing at least one hydrophilic unit of the type of an olefinically unsaturated carboxylic acid and at least one hydrophobic unit exclusively of the type of an alkyl(C₁₋₃₀) ester of an unsaturated carboxylic acid, and methacrylic acid/methylacrylate/dimethyl-meta-isopropenylbenzyl isocyanate copolymers of an ethoxylated alcohol.

12. The cosmetic composition according to one of claims 1 to 8, **characterized in that** the cationic amphiphilic polymers are selected from quaternized cellulose derivatives and polyacrylates having aminated side chains.

13. The cosmetic composition according to one of claims 1 to 8, **characterized in that** the amphoteric amphiphilic polymers containing at least one fatty chain are selected from copolymers of methacrylamidopropyltrimethylammonium chloride/acrylic acid/alkyl(C₁₀₋₃₀) methacrylate.

14. The cosmetic composition according to one of claims 1 to 13, **characterized in that** it contains the thickener(s) in a total amount of from 0.001 to 20 wt.%, preferably 0.01 to 15 wt.%, more preferably 0.1 to 10 wt.% and in particular 0.25 to 5 wt.%, based on the total weight of the composition.

15. A method for treating keratin fibers, **characterized in that** a hair treatment agent according to one of claims 1 to 14 is applied to the keratin fibers and rinsed out again after a contact time of from a few seconds up to 45 minutes.

16. The use of a composition according to one of claims 1 to 15
- for conditioning keratin substances and/or
- for improving looseness, smoothness, shine and/or combability and for facilitating hairdressing of keratin substances and/or
- for improving remanence of the conditioning effect when washing the hair and/or
- for improving the wet and dry combability and/or
- for improving the shine and/or
- for improving the moisture balance of keratin fibers and/or
- for protecting the keratin fibers from oxidative damage and/or
- for preventing keratin fibers from becoming greasy again and/or
- for increasing the washing resistance of colored keratin fibers.

## Revendications

1. Composition cosmétique contenant, dans un milieu cosmétiquement acceptable,
(a) au moins un épaississant sélectionnés parmi les épaississants associatifs
(b) au moins un organopolysiloxane à terminaison hydroxy de la formule générale (I), 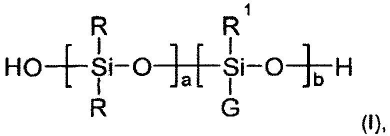 dans laquelle
- R est un radical hydrocarboné monovalent non substitué ou substitué par un halogène et ayant 1 à 20 atomes de carbone,
- R¹ est un radical hydrocarboné monovalent non substitué ou substitué par un halogène et ayant 1 à 20 atomes de carbone, -OR⁴ ou -OH,
- R⁴ est un radical alkyle ayant 1 à 6 atomes de carbone,
- G est un groupe de la formule générale (II) 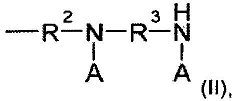
dans laquelle
- R², R³ sont indépendamment un radical hydrocarboné divalent ayant 1 à 6 atomes de carbone, des unités -CH₂ non adjacentes pouvant être remplacées par unités sélectionnées parmi -C(=O)-, -O- et -S-,
- A est R⁵-C(=O)-,
- R⁵ est un radical alkyle ayant 1 à 20 atomes de carbone,
- a représente des valeurs entières de 100 à 1500 et
- b représente des valeurs entières au moins égales à 1.

2. Composition cosmétique selon la revendication 1, **caractérisée** en ce quelle contient au moins un organopolysiloxane à terminaison hydroxy de la formule générale (I) dans laquelle R est choisi parmi les radicaux méthyle, éthyle, n-propyle, isopropyle, n-butyle, isobutyle, sec-butyle, tert-butyle, n-pentyle, n-hexyle, R représentant de façon très préférée un méthyle.

3. Composition cosmétique selon l'une des revendications 1 ou 2, **caractérisée en ce qu'**elle contient au moins un organopolysiloxane à terminaison hydroxy de la formule générale (I) dans laquelle les radicaux R², R³ sont choisis parmi éthylène, n-propylène, iso-butylène ou n-butylène.

4. Composition cosmétique selon l'une des revendications 1 à 3, **caractérisée** parce qu'il contient au moins un organopolysiloxane à terminaison hydroxy de la formule générale (I) dans laquelle R⁵ est choisi parmi les radicaux méthyle, éthyle, n-propyle, isopropyle, n-butyle, isobutyle, sec-butyle, tert-butyle, n-pentyle, n-hexyle, R⁵ représentant de façon très préférée un radical méthyle ou éthyle.

5. Composition cosmétique selon l'une des revendications 1 à 4, **caractérisée en ce qu'**elle contient, sur la base de son poids, de 0,00001 à 10% en poids, de préférence de 0,0001 à 7,5% en poids, de manière particulièrement préférée de 0,001 à 5% en poids, plus préférablement de 0,01 à 3% en poids et en particulier de 0,1 à 1% en poids, d'une de plusieurs organopolysiloxanes à terminaison hydroxy de la formule générale (I).

6. Composition cosmétique selon l'une des revendications 1 à 5, **caractérisée en ce qu'**elle contient, sur la base de son poids, de 0,00001 à 5% en poids, de préférence de 0,0001 à 3,5% en poids, de manière particulièrement préférée de 0,001 à 2% en poids, plus préférablement de 0,01 à 1% en poids et en particulier de 0,1 à 0,5% en poids, de Tridécanol éthoxylé ramifié (nom INCl : Trideceth-5) ou α-iso-tridécyle-ω-hydroxypolyglycoléther (nom INCl : Trideceth-10) ou des mélanges de ceux-ci.

7. Composition cosmétique selon l'une des revendications 1 à 6, **caractérisée en ce que** la masse moléculaire moyenne en poids de l'organopolysiloxane à terminaison hydroxy de la formule générale (I), est dans la gamme de 2000 à 1000000 gmol⁻¹, de préférence dans la gamme de 5000 à 200000 gmol⁻¹.

8. Composition cosmétique selon l'une des revendications 1 à 7, **caractérisée en ce que** l'organopolysiloxane à terminaison hydroxy de la formule générale (I) se présente sous la forme d'une émulsion huile-dans-eau dans laquelle la taille moyenne en nombre des particules de silicone dans l'émulsion est dans la gamme de 3 à 500 nm, de préférence dans la gamme de 5 à 60 nm.

9. Composition cosmétique selon l'une des revendications 1 à 8, **caractérisée en ce que** l'épaississant associatif est un polymère associatif qui est choisi parmi
(i) les polymères amphiphiles non-ioniques contenant au moins une chaîne grasse et au moins une unité hydrophile ;
(ii) les polymères amphiphiles anioniques contenant au moins une unité hydrophile et au moins une unité à chaîne grasse ;
(iii) les polymères amphiphiles cationiques contenant au moins une unité hydrophile et au moins une unité à chaîne grasse ;
(iv) les polymères amphiphiles amphotères contenant au moins une unité hydrophile et au moins une unité à chaîne grasse,
les chaînes grasses comportant de 10 à 30 atomes de carbone.

10. Composition cosmétique selon les revendications 1 à 8, **caractérisée en ce que** les polymères amphiphiles non-ioniques contenant au moins une chaîne grasse et au moins une unité hydrophile, sont choisies de préférence parmi
(1) les celluloses modifiées par des groupes comportant au moins une chaîne grasse ;
(2) des composés hydroxypropylguar modifiés par des groupes comportant au moins une chaîne grasse ;
(3) des uréthanpolyéthers contenant au moins une chaîne grasse tels que des groupes alkyle ou alcényle ayant 10 à 30 atomes de carbone ;
(4) des copolymères de vinylpyrrolidone et de monomères hydrophobes à chaîne grasse ;
(5) des copolymères de méthacrylates d'alkyle en C₁ à C₆ ou d'acrylates d'alkyle en C₁ à C₆ et de monomères amphiphiles contenant au moins une chaîne grasse ;
(6) des copolymères de méthacrylates ou d'acrylates hydrophiles et de monomères hydrophobes contenant au moins une chaîne grasse.

11. Composition cosmétique selon l'une des revendications 1 à 8, **caractérisée en ce que** les polymères amphiphiles anioniques contenant au moins une unité hydrophile et au moins une unité à chaîne grasse sont choisis parmi les composés contenant au moins une unité éther d'allyle à chaîne grasse et au moins une unité hydrophile obtenue à partir d'un monomère anionique éthyléniquement insaturé, les composés contenant au moins une unité hydrophile du type acide carboxylique oléfiniquement insaturé et au moins une unité hydrophobe exclusivement du type d'un ester d'alkyle en C₁ à C₃₀ d'un acide carboxylique insaturé et des copolymères acide méthacrylique/acrylate de méthyle/cyanate de diméthyl*méta*-isopropényl-benzyle.

12. Composition cosmétique selon l'une des revendications 1 à 8, **caractérisée en ce que** les polymères amphiphiles cationiques sont choisis parmi les dérivés de cellulose quaternisés et les polyacrylates à chaînes latérales aminées.

13. Composition cosmétique selon l'une des revendications précédentes 1 à 8, **caractérisée en ce que** les polymères amphiphiles amphotères contenant au moins une chaîne grasse sont choisis parmi les copolymères de chlorure de méthacrylamidopropyltriméthylammonium/acide acrylique/méthacrylate d'alkyle en C₁₀ à C₃₀.

14. Composition cosmétique selon l'une des revendications 1 à 13, **caractérisée en ce qu'**elle contient la ou les épaississants dans une quantité totale de 0,001 à 20% en poids, de préférence de 0,01 à 15% en poids, plus préférablement de 0,1 à 10% en poids et en particulier de 0,25 à 5% en poids, par rapport au poids total de la composition.

15. Procédé de traitement de fibres de kératine, **caractérisée en ce qu'**une composition de traitement capillaire selon l'une des revendications 1 à 14 est appliquée sur les fibres de kératine et est éliminé par rinçage après un temps d'action de quelques secondes à 45 minutes.

16. Utilisation d'une composition selon l'une des revendications 1 à 15
- pour conditionner des substances kératiniques et/ou
- pour améliorer le relâchement, la douceur, la brillance et la capacité de peignage et pour faciliter le coiffage de substances kératiniques et/ou
- pour améliorer la rémanence de l'effet de conditionnement pendant le lavage des cheveux et/ou
- pour améliorer la capacité de peignage sur cheveux secs et mouillés et/ou
- pour améliorer la brillance et/ou
- pour améliorer la teneur en ,humidité des fibres de kératine et/ou
- pour protéger les des fibres de kératine contre l'oxydation et/ou
- pour empêcher le regraissage des fibres de kératine et/ou
- pour augmenter la résistance au lavage de fibres de kératine.
